# EUROPEAN PATENT SPECIFICATION

(11) **EP 0 502 664 B1**
(45) Date of publication and mention of the grant of the patent: **10.04.1996**
(21) Application number: 92301712.3
(22) Date of filing: 28.02.1992
(51) Int. Cl.: A61M 25/00

(54) **Site-selective reinforced catheter and methods of manufacturing the reinforced catheter**
Lagenselektiver, verstärkter Katheter und Verfaren für die Herstellung des verstärkten Katheters
Cathéter renforcé sélectif de position et procédé pour sa fabrication

(30) Priority: 07.03.1991 US 665787
(43) Date of publication of application: 09.09.1992
(73) Proprietor: C.R. BARD, INC., Murray Hill New Jersey 07974 (US)
(72) Inventor: Moorehead, H. Robert, Salt Lake City Utah 84121 (US)
(74) Representative: Lehn, Werner, Dipl.-Ing.

(56) References cited:
- EP-A- 0 144 629
- EP-A- 0 440 992
- WO-A-89/09079
- US-A- 4 551 292

## Description

The present invention relates to methods and apparatus for the site-selective reinforcement for a catheter which is subjected to potentially damaging stresses due to body movement. In particular, the present invention relates to a method and apparatus for the reinforcement of the portion of a soft, medical grade, silicone rubber catheter which, whether by inadvertence or design, becomes disposed in the compressed area between the clavicle and first rib of a patient when the catheter is installed in the subclavian vein.

Patients undergoing long-term intravenous therapy have benefited from indwelling central venous access catheters. These catheters have proven very effective for infusing chemotherapeutical drugs and antibiotics, and for providing access for withdrawing blood. Nonetheless, since the introduction of these catheters, a relatively low, but constant, level of usage complications has occurred with the catheters. Complications related to the method of catheter insertions through the percutaneous subclavian route have been described in a number of medical journals.

The types of complications vary, but the results may be generalized. Reports indicate a partial separation of the implanted catheters that subsequently leads to total catheter separation and possible embolization. It is an obvious health risk to a patient should embolization occur near the heart.

As the axillary vein becomes the subclavian vein, it travels in a very narrow triangular space bounded by the clavicle and first rib anteriorly and the anterior scalene muscle posteriorly. Catheters inserted using the percutaneous subclavian route pass medial to the subclavian vein in an extremely narrow space bounded by the costo-clavicular ligament in the angle between the first rib and clavicle anteriorly and the subclavian vein posteriorly. It is at this angle (while outside the vein) where a catheter implanted by the subclavian route can become caught in a pincher-like pressure from movement over time involving the shoulder and clavicle. Due to this problem, this area is commonly referred to as the "pinch-off area."

Research has indicated that the probable cause of most reported partial separations, and total separations of implanted catheters is due to long-standing compression of the catheter at the narrow space between the clavicle and first rib -- i.e., the "pinch-off area." The repeated compressing, grinding, and rubbing of the exterior surface of the implanted catheter by the pincher-like pressure from the movement of the shoulder and clavicle causes the weakening that leads to partial or total catheter separation.

Partial separation of the catheter can lead to various complications. The most important of which is that an eventual hole in the catheter wall, caused by the pincher action described above, will allow extravasation of infusates. Some of these infusates are extremely toxic, and have caused surgery to be performed to remove pockets of resulting dead tissue around the leak site.

Total separation of the catheter into two portions can also lead to various complications. The partial separation of the catheter which will eventually lead to total separation due to continued compression, can lead to embolization. The pincher action totally separates the catheter, and with blood flow to propel it, the distal end of the catheter almost always ends in the pulmonary vessels leading from the right ventricle to the lungs. Surgical procedures are warranted by such an event and results in increased trauma to a patient both mentally and physically.

For obvious reasons, a catheter must, for the most part, be compliant due to its passage throughout relatively soft layers of skin. Flexibility by the catheter prevents unnecessary irritation of tissue layers. It is, however, this compliancy which promulgates the complications related to the method of catheter insertions through the percutaneous subclavian route.

Solutions to the problem do exist. It has been noted that the compression of the catheter is alleviated by having patients lie supine or with the arm and shoulder slightly raised. For obvious reasons, it cannot be suggested that a patient maintain such a position indefinitely. Such a procedure requires a major deviation from currently existing standard practices and requires significant and difficult handling by medical personnel. Hence, for long-term indwelling catheters, this is not an adequate solution.

Alternately, it has been proposed to increase the thickness of the catheter wall or to interweave metallic substances in the wall of the catheter to counteract the regions of stress imposed thereon by the body of the patient. Unfortunately, if the catheter is noncompliant and relatively stiff so that it cannot undergo excessive flexing, the movement of the catheter is likely to irritate or damage the relatively soft layers of tissue which surround the catheter. Again, this is an inadequate response for long-term indwelling catheters where the thickness of metal substances causes an unacceptable irritation or damage to adjacent tissues. It has also been proposed to mold a second layer of a pliable material over the entire length of a catheter which would increase the torsional rigidity of the catheter. But for reasons previously set forth, such a procedure would result in a noncompliant and relatively stiff catheter which could irritate bodily layers, and erode areas of vital organs of the body when a catheter comes in contact with such organs. Also, this procedure is not site-selective and the area chosen for reinforcement could not be readily converted into an area which would not require reinforcement. These solutions are considered to represent the closest prior art with regard to the present invention.

A need, therefore, exists in the art for an indwelling central venous access catheter which will not be susceptible to compressive forces experienced by such a catheter implanted via the percutaneous subclavian route. The catheter should not risk embolization to a patient by the partial separation, or total separation by the catheter.

Additionally, the catheter should have the capacity to be placed and used without a major deviation from currently existing standard practices. The catheter should also be easy to handle and manipulate by medical personnel to enable the catheter's placement and use.

Further, the reinforcing sleeve of such a catheter should be placed so as not to irritate bodily tissues, the reinforced catheter should be soft enough to easily follow the normal path of a catheter as well as being capable of preventing partial separation to allow for the extravasation of or total separation of a catheter into two portions.

The present invention seeks to resolve problems incidental to catheters, particularly percutaneously placed catheters, subjected to potentially damaging stresses due to body movement. More specifically, the apparatus and method of this invention constitute an important advance in the reinforcement of the portion of a soft, medical grade, silicone rubber catheter which becomes disposed and sometimes separated in the compressed area between the clavicle and first rib of a patient when the catheter is implanted in the subclavian vein.

One object of the present invention is to provide an apparatus and method for reinforcing a percutaneously placed catheter disposed in an area subject to damaging stresses due to body movement which will reduce susceptibility of catheters implanted via the percutaneous subclavian route to the damaging effects of such compressive forces.

Also, it is an object of the present invention to provide an apparatus and method for reinforcing a percutaneously placed catheter disposed in an area subject to damaging stresses due to body movement which can prevent embolization of the implanted catheter and the associated risks to the patient by eliminating the risk of partial separation, or total separation of the catheter.

Additionally, it is an object of the present invention to provide an apparatus and method for reinforcing a percutaneously placed catheter disposed in an area subject to damaging stresses due to body movement which will have the capacity to place and use the catheter without a major deviation from currently existing standard practices.

Another object of the present invention is to provide an apparatus and method for reinforcing a percutaneously placed catheter disposed in an area subject to damaging stresses due to body movement which will allow the insertion of a reinforcing sleeve which will be soft enough to easily follow the normal path of a catheter as well as being able to prevent partial separation of the catheter to allow for the extravasation of or the total separation of the catheter into two portions.

Still a further object of the present invention is to provide an apparatus and method for reinforcing a percutaneously placed catheter disposed in an area subject to damaging stresses due to body movement which is easy to handle and manipulate by medical personnel.

Additional objects and advantages of the invention will be set forth in the description which follows, and in part will be obvious from the description, or may be learned by the practice of the invention. The objects and advantages of the invention may be realized and obtained by means of the instruments and combinations particularly pointed out in the appended claims.

Briefly summarized, the foregoing objects and advantages are realized by the apparatus and method of the present invention as defined in Claims 1 and 15 respectively, wherein a reinforced catheter is placed in an area subject to potentially damaging stresses due to body movement by methods which do not radically depart from currently existing standard practices for placing the catheter. Although the reinforced catheter experiences damaging stresses, the reinforced catheter does not partially separate, or totally separate, and thereby prevents the allowance for the extravasation of or embolization of a catheter section in the body of the patient near the heart.

The reinforced catheter may comprise a non-metallic inner tubular sleeve made wholly of one type of compliable material that is inserted selectively within a catheter. In one embodiment, the tubular sleeve has chamfered ends which conforms the tubular sleeve to the configuration of the inside of the catheter it resides within to allow materials to travel unhindered through the tubular sleeve.

The reinforcing inner sleeve is selectively positioned at the point corresponding to the regions of stress imposed on the catheter by the body of the patient e.g., in the case of long-term indwelling central venous access catheters that can become caught in the pincher-like pressure in the subclavian route in between the first rib and clavicle. It should be pointed out, however, that the foregoing example is provided for clarification, and is not meant to limit the scope of areas associated with a patient that the reinforcing catheter may be employed.

According to the present invention, the reinforcing inner sleeve is positioned within the catheter lumen. The reason for this structural arrangement, the possibility of infusates passing through partial separations in the catheter wall is prevented. Likewise, the possibility of total catheter separation leading to embolization is prevented.

In spite of any degradation and separation experienced by the outside of the catheter from any inwardly directed compressive forces, the reinforcing inner sleeve remains intact. This is due to the fact that the outer diameter of the reinforcing inner sleeve is slightly larger than the inner diameter of the catheter. Thus, when the reinforcing inner sleeve resides within the catheter, the catheter presses and frictionally engages the exterior of the reinforcing inner sleeve.

In fact, the uninjured reinforcing inner sleeve will even continue to maintain a connection between severed portions of a damaged catheter in order to prevent the complications arising in a patient from embolization because the severed portions will continue to remain in frictional engagement with the reinforced sleeve. Thus, the reinforced catheter can be used for long periods of time even if there is a partial separation or total separation of the catheter wall.

Two methods are disclosed herein for the manufacture of the reinforced catheter: (1) the "chemical soak, " and (2) the "pressurized gas" methods. Of course, other additional methods may be available.

In the "chemical soak" method, the catheter is immersed in a material, such as freon, which causes the silicone, of which such catheters are often constructed, to swell. The sleeve, which typically is made of polyurethane is placed on a rod abutted against an encircling washer. The rod and sleeve are then advanced into the catheter to the position desired, whereupon the rod is withdrawn. Thereafter, the portions of the catheter exposed to freon are dried, and the catheter returns to its original size. The result is that the reinforcing inner sleeve is trapped.

The "pressurized gas" method is the presently preferred method of assembly. Air at a high pressure and controlled volume is utilized to partially inflate the catheter and at the same time to provide a nontoxic, low-friction interface between the polyurethane sleeve and the inner surface of the catheter. According to this method, the catheter is softclamped at a position intermediate the desired reinforcement site and the distal openings to the lumen therein.

The reinforcing inner sleeve is placed on a hollow rod, such as a needle cannula abutted against an encircling push ring. The end of the hollow push rod remote from the reinforcing inner sleeve is connected to a low-volume, high pressure air source connected to the air line. The end of the push rod with the reinforcing inner sleeve mounted thereon is entered to and advanced along the lumen of the catheter from the open proximal end thereof. The pressurized air passing through the hollow rod inflates and slightly enlarges the catheter and escapes therefrom between the advancing reinforcing inner sleeve and the inner wall of the catheter. As a result, the sleeve and push rod are easily advanced into position, whereupon the rod is withdrawn from the sleeve and the catheter.

In order that the manner in which the above-recited and other advantages and objects of the invention are obtained, a more particular description of the invention briefly described above will be rendered by reference to specific embodiments thereof which are illustrated in the appended drawings. Understanding that these drawings depict only typical embodiments of the invention and are therefore not to be considered as limiting of its scope, the invention will be described with additional specificity and detail through the use of the accompanying drawings in which:
Figure 1 is a perspective view of the preferred embodiment of the present invention as an apparatus.
Figure 1A is a planar side view of a catheter illustrating the configuration of the catheter when it experiences compressive forces.
Figure 1B is a planar side view of a catheter illustrating the configuration of the catheter totally separated due to compressive forces.
Figure 2 is a perspective view of the preferred embodiment of the present invention by the "chemical soak" method as the catheter is treated with a chemical.
Figure 3 is a perspective view of the preferred embodiment of the present invention by the "chemical soak" method displaying the catheter after it has been treated with a chemical.
Figure 4 is a planar side view of the preferred embodiment of the present invention by the "chemical soak" method displaying the reinforcing inner sleeve being inserted along the portion of the enlarged catheter that had been treated with a chemical.
Figure 5 is a planar side view of the preferred embodiment of the present invention by the "chemical soak" method displaying the rod being retracted from the catheter lumen after insertion of the reinforcing inner sleeve.
Figure 6 is a planar side view of the preferred embodiment of the present invention by the "chemical soak" method displaying the reinforced catheter after the catheter has been dried.
Figure 7 is a perspective view of the preferred embodiment of the present invention by the "pressurized gas" method displaying the reinforcing inner sleeve which is connected to an air pump.
Figure 8 is a planar side view of the preferred embodiment of the present invention by the "pressurized gas" method displaying the enlargement of the catheter lumen by pressurized air as the reinforcing inner sleeve is inserted.
Figure 9 is a planar side view of the preferred embodiment of the present invention by the "pressurized gas" method displaying the insertion route of the reinforcing inner sleeve as it is guided by the hollow push rod.
Figure 10 is a planar side view of the preferred embodiment of the present invention by the "pressurized gas" method displaying the reinforced catheter after the hollow push rod has been withdrawn and illustrating the conformation of the reinforcing catheter as it is subjected to compressive forces.

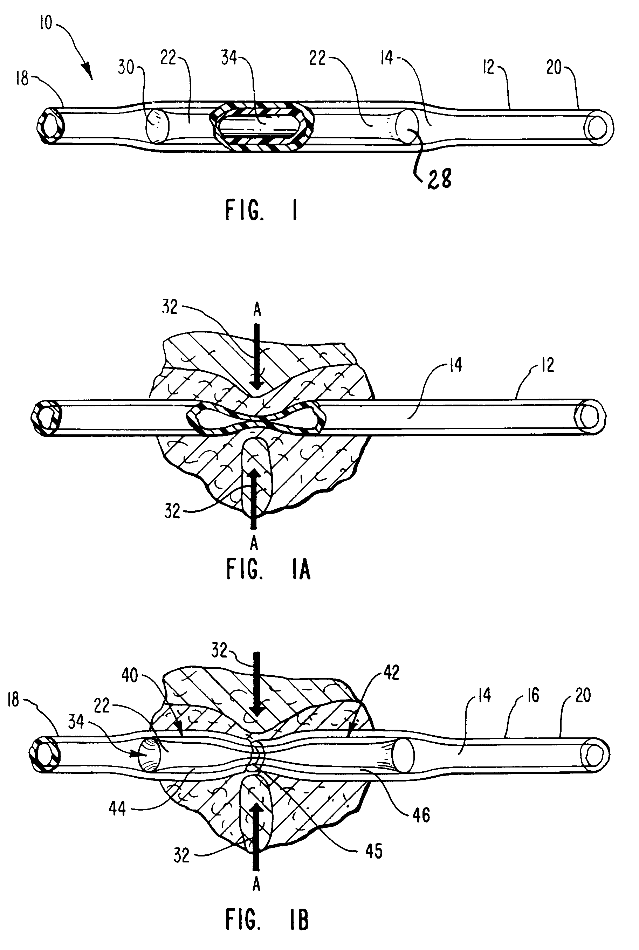

The present invention can be best understood by reference to the drawings, wherein like parts are designated with like numerals throughout. The present invention as it pertains to the apparatus can be understood with reference to Figure 1. Figure 1 illustrates the apparatus disclosed in the present invention in its preferred embodiment.

Figure 1 illustrates an apparatus which site-selectively reinforces a region of a catheter, the apparatus commonly referred to in this application as the reinforced catheter 10. Reinforced catheter 10 comprises a catheter 12 made of a compliant medical grade material with a longitudinal lumen 14 extending through a substantial length of catheter 12. A region of catheter 12 can be subjected to compressive forces if catheter 12 is placed in the vessel of a patient (not shown).

In the preferred embodiment of the present invention, the choice of material catheter 12 is made from is silicone rubber. This type of material is preferred in light of its biocompatible, compliable nature. Also, ease of catheters, made from silicone rubber, on bodily tissues as the catheters are inserted into a body, makes this choice of material ideal. This type of material, however, is discussed as an example of what catheters can be made from; the present invention is not limited to this choice of material.

In the present invention it is also preferred that catheter 12 be an indwelling central venous access catheter. Catheter 12 is typically percutaneously implanted within a patient for a time period which can range from about ten minutes to about two years. Nevertheless, the present invention includes all types of catheters, and catheter 12 is not required to be an indwelling central venous access catheter.

In addition, catheter 12 may have a two-way valve, but again this is not required. Two-way valve is located at the distal end 18 of catheter 12. Proximal end 20 of catheter 12 has only a general opening. The edges of the two-way valve remain in abutment and closed in a normal state. The two-way valve, however, responds to pressure differentials and will disengage to create an opening so that materials may pass through the two-way valve.

Further, catheter 12 can have a single lumen or catheter 12 can have more than one lumen. As illustrated in Figure 1, catheter 12 has only a single lumen 14.

As an example of the compressive forces mentioned, it is known that if catheter 12 is implanted in the subclavian vein of a patient, catheter 12 travels in a very narrow triangular space bounded by the clavicle and first rib anteriorly and the anterior scalene muscle posteriorly. Catheter 12 inserted using the percutaneous subclavian route pass medial to the subclavian vein in an extremely narrow space bounded by the costo-clavicular ligament in the angle between the first rib and clavicle anteriorly and the subclavian vein posteriorly. It is at this angle (while outside the vein) where catheter 12 implanted by the subclavian route can become caught in a pincher-like pressure from movement over time involving the shoulder and clavicle.

According to one embodiment of the present invention, in order to reinforce the regions of catheter 12 found in areas generating compressive forces, means are provided for site selectively reinforcing the aforesaid region of catheter 12 where the compressive forces are applied against catheter 12. The means are positioned within lumen 14 of catheter 12.

As shown by way of example and not limitation, a nonmetallic inner sleeve 22 having a proximal end 28 and a distal end 30 is inserted site-selectively within catheter 12. Nonmetallic inner sleeve 22 is tubular in structure. This construction is important so that nonmetallic inner sleeve 22 does not present an obstacle to the flow of fluids passing through lumen 14 of catheter 12. The passage of these fluids is critical to a patient. Nonmetallic inner sleeve 22 is capable of preventing catheter 12 from becoming partially or totally separated due to compressive forces generated by the body of a patient.

Also enabling the generally constant flow of a fluid through lumen 14 are the structural features of nonmetallic inner sleeve 22. In one embodiment of the present invention, proximal end 28 and distal end 30 of nonmetallic inner sleeve 22 have chamfered edges. The chamfered edges conform nonmetallic inner sleeve 22 to the configuration of the inside of catheter 12 to allow materials to travel unhindered through nonmetallic inner sleeve 22.

Nonmetallic inner sleeve 22 can be comprised wholly of a single type of material or of different substances. In the preferred embodiment of the present invention, nonmetallic inner sleeve 22 is comprised of polyurethane. This material is resistant to separation by compressive forces and maintains a conformational integrity. In other embodiments, nonmetallic inner sleeve has been comprised of tetrafluoroethylene or fluorinated ethylene propylene. The present invention, however, is not limited to these constructions.

Also, nonmetallic inner sleeve 22 has an outer diameter greater than the inner diameter of catheter 12. In this way, when nonmetallic inner sleeve resides in lumen 14 of catheter 12, the inner walls of catheter 12 frictionally engages the exterior of nonmetallic inner sleeve. It is this frictional engagement which maintains the position of nonmetallic inner sleeve 22 relative to catheter 12.

Also, because of the frictional engagement between nonmetallic inner sleeve 22 and catheter 12, embolization of a portion of catheter 12 in a patient is prevented. Should catheter 12 partially separate or totally separate because of the previously mentioned compressive forces, the frictional engagement prevents the allowance for the extravasation of infusates or the separated portion of catheter 12 from separating from nonmetallic inner sleeve 22. Therefore, nonmetallic inner sleeve 22 maintains a constant connection between fractured or separated portions of catheter 12.

It is important to reemphasize that nonmetallic inner sleeve 22 is disposed within lumen 14 of catheter 12. At first glance, it would seem to be easier to have catheter 12 disposed within nonmetallic inner sleeve 22 due to the larger outer diameter of nonmetallic inner sleeve 22 than the inner diameter of catheter 12. Positioning nonmetallic inner sleeve 22 could be done with relative ease by stretching the ends of catheter 12 slightly to sufficiently reduce the outer diameter of catheter 12 so that nonmetallic inner sleeve 22 could be slid into position about catheter 12.

This structural arrangement is also one of the reasons why this solution is undesirable. Nonmetallic inner sleeve 22 does not prevent stresses from being transmitted to catheter 12 when the two are disposed adjacent each other. Thus, should the reinforced catheter in the foregoing structural arrangement be subjected to compressive forces, catheter 12 will still partially or totally separate. At this critical point, if catheter 12 is removed from a patient (the most common removal procedure is to pull on the catheter and break a Dacron cuff loose from a patient's tissue) and catheter 12 is partially or totally separated, catheter 12 will stretch and pull out of nonmetallic inner sleeve 22.

Due to the foregoing, nonmetallic inner sleeve 22 and the distal end of catheter 12 are left within the patient. As previously discussed, once the compressive forces about nonmetallic inner sleeve 22 are relieved by body movement, these portions can be quickly carried into the heart. The resulting embolus can cause heart arrhythmias, and be a focal point for very serious infection in the cardiovascular system.

The structural arrangement of the present invention prevents any such dangers from occurring to a patient. With nonmetallic inner sleeve 22 disposed within catheter 12, totally separated sections of catheter 12 cannot be pulled off of nonmetallic inner sleeve 22. This is due to the elongation and neck down of catheter 12, making catheter 12 even tighter on nonmetallic inner sleeve 22. Restriction on catheter 12, and not on nonmetallic inner sleeve 22 results in an accordion effect on catheter 12 over nonmetallic inner sleeve 22 which raises the frictional interface between the catheter 12 and nonmetallic inner sleeve 22.

In the preferred embodiment of the present invention the inner diameter of catheter 12 is in the range from about 1,40 to 1,70 mm (.055 to .066 inches) and the outer diameter of nonmetallic inner sleeve 22 is in the range from about 1,80 to 1,96 mm (.071 to .077 inches). In the most preferred embodiment, the outer diameter of nonmetallic inner sleeve 22 needs to be large enough to frictionally interface with the inner diameter of catheter 12. Nevertheless, the present invention is not limited to these dimensions.

In one embodiment of the present invention, nonmetallic inner sleeve 22 is positioned in the range from about 25.4 mm to about 610 mm (about one inch to about two feet) from distal end 18 of catheter 12. In the most preferred embodiment, however, nonmetallic inner sleeve 22 is positioned about 38 mm (about 1.5 inches) from distal end 18 of catheter 12.

The length of nonmetallic inner sleeve 22 is in one embodiment of the present invention in the range from about 200 mm to about 216 mm (from about 8 inches to about 8.5 inches). In the most preferred embodiment, however, nonmetallic inner sleeve 22 is about 210 mm (about 8.25 inches) in length.

Figure 1A illustrates the effect compressive forces have on a prior art catheter that is not reinforced. Compressive forces 32 are directed inwardly as indicated by arrow A. In this way, the conformation of catheter 12 in Figure 1A is distorted. Repeated infliction of compressive forces 32 on the outer surface of catheter 12 will wear down the body of catheter 12. Eventually, the body of catheter 12 becomes brittle and can partially separate or totally separate.

Figure 1 illustrates how reinforcing catheter 10 reacts to compressive forces 32. Figure 1 contains a cut-away view of the lumen 34 of nonmetallic inner sleeve 22 which changes its conformation when compressive forces 32 engage the body of reinforced catheter 10. Likewise, lumen 14 of catheter 12 changes its conformation. As it will be more thoroughly discussed at a future point, however, catheter 12 remains in frictional engagement with nonmetallic inner sleeve 22 even if compressive forces 32 partially or totally separate catheter 12.

Because nonmetallic inner sleeve 22 is slightly larger than lumen 14 of catheter 12, the body of catheter 12 must be expanded to accept nonmetallic inner sleeve 22. Once nonmetallic inner sleeve 22 is positioned within catheter 12, the body of catheter 12 attempts to regain its non-expanded conformation and presses inwardly on nonmetallic inner sleeve 22. This result has the effect of keeping nonmetallic inner sleeve 22 in place within lumen 14 of catheter 12. In this way, once positioned to counteract generated compressive forces 32, nonmetallic inner sleeve 22 will stay in the same place so as not to slide or move.

An additional benefit from the body of catheter 12 pressing against nonmetallic inner sleeve 22 is that if the body of catheter 12 around nonmetallic inner sleeve 22 is worn away by the continued bombardment of compressive forces 32, nonmetallic inner sleeve 22 continues to serve as a connective body between the severed catheter body portions. This benefit is better illustrated by reference to Figure 1B, wherein catheter 12 resides in the area of a patient between the clavicle and the first rib which generates compressive forces 32. Due to compressive forces 32, catheter 12 has become totally separated.

In Figure 1B, catheter 12 is separated into a first section 40 and a second section 42 by compressive forces 32. The tubular structure of nonmetallic inner sleeve 22 extends the communication between the lumen 44 of first section 40 and the lumen 46 of second section 42 of separated catheter 12. The separated area 45 can be fairly close together as illustrated in Figure 1B; however, this may not occur in all instances.

Connection between first section 40 and second section 42 of severed catheter 12 results because the outer diameter of nonmetallic inner sleeve 22 is slightly larger than the inner diameter of catheter 12. Thus, when nonmetallic inner sleeve 22 resides within lumen 14 of catheter 12, catheter 12 presses and frictionally engages the exterior of nonmetallic inner sleeve 22. Thereafter, should separation of the intravascular catheter wall occur, first section 40 and second section 42 of catheter 12 remain in frictional engagement with nonmetallic inner sleeve 22 which continues to provide the communication between lumen 44 of first section 40 and lumen 46 of second section 42 of separated catheter 12.

Selective site reinforcement of an implanted catheter to compensate for specific areas of compressive stress imposed by the body of a patient should not be confused with the binding of a catheter along most or all of its length to increase the torsional rigidity of the catheter. Compression involves the squeezing together of a section of the catheter. Catheter separation and embolization occur as a result of long-term compression imposed on the body of the catheter.

Torsion, on the other hand, involves the twisting or wrenching of the catheter by the exertion of forces tending to turn one end or part about a longitudinal axis while the other is held fast or turned in the opposite direction. Any twisting or wrenching of a catheter can only result in the damage to sensitive bodily layers of a patient through which a catheter resides. Under the circumstances of the present invention, it is compressive forces which the reinforced catheter experiences and is primarily directed to correct. Nevertheless, the area of site-selective reinforcement would also be torsionally resistant.

It is important to note that catheter 12 is preformed in relation to nonmetallic inner sleeve 22. Various individuals have attempted to increase the torsional rigidity of catheters by taking a tubular structure and forming a catheter body around the tubular structure. The dual-body structure of the resulting catheter by this "after-forming" procedure increases the torsional rigidity of the structure. The tubular structure of the after-formed catheter body has been comprised of such rigid materials such as metals.

In the present invention, nonmetallic inner sleeve 22 is inserted within a catheter which has already been formed, and therefore reinforced catheter 10 is quite different from the catheter created by the after-formed procedure. Nonmetallic inner sleeve 22 is site-selectively positioned within catheter 12 to reinforce against compressive forces 32 unlike the after-formed catheter. Also, nonmetallic inner sleeve 22 substantially increases the torsional rigidity of catheter 12 as would an after-formed catheter.

Moreover, the differences between an after-formed catheter and nonmetallic inner sleeve 22 can be shown by a further examination of their methods of manufacture. The after-formed catheter is formed by molding an outer layer around a tubular structure as previously discussed.

The present application discloses two separate methods that have been used to manufacture reinforced catheter 10: (1) the "chemical soak," and (2) the "pressurized gas" methods. Both these methods, as will be seen, vary greatly from the method of manufacturing the after-formed catheter.

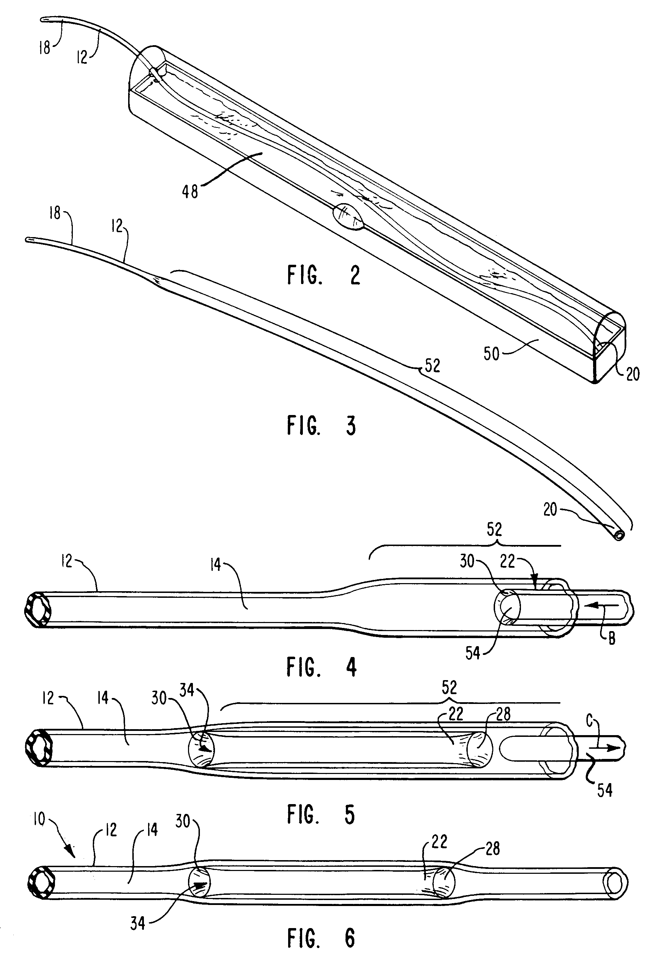

The present invention as it pertains to the chemical soak method can be understood with reference to Figure 2. Figure 2 illustrates this method for site-selectively reinforcing a portion of catheter 12 in its preferred embodiment. The first step of the chemical soak method, involves exposing a length of catheter 12 to a chemical compound which causes the exposed portion of catheter 12 to swell.

This chemical treatment step is important because in this way, lumen 14 of catheter 12, which before the chemical treatment was of a diameter smaller than nonmetallic inner sleeve 22, is expanded to a diameter greater than nonmetallic inner sleeve 22. This result allows nonmetallic inner sleeve 22 to be slidably engaged within lumen 14 of catheter 12.

Because it is implicit in the invention that part of catheter 12 be reinforced against compressive forces 32, those portions of catheter 12 must therefore be immersed in the chemical and expanded so nonmetallic inner sleeve 22 can be placed within to resist compressive forces 32. As it can be seen in Figure 2, those portions of catheter 12 through which nonmetallic inner sleeve 22 will be slidably engaged need also be immersed in the chemical in order for nonmetallic inner sleeve 22 to travel from the inserted end to the siteselective position. The remainder of catheter 12 can extend into the area surrounding the area where the chemical solution is kept.

It is important to note that because nonmetallic inner sleeve 22 can be inserted in any expanded opening, and catheter 12 has proximal end 20 and distal end 18, an option exists for nonmetallic inner sleeve 22 to be inserted through an opening other than at distal end 18 of catheter 12. The distal end of a catheter may be structurally sensitive, e.g**.** if a two-way valve exists. In the preferred embodiment of the present invention, therefore, insertion of nonmetallic inner sleeve 22 through expanded proximal end 20 of catheter 12 alleviates any potential dangers were nonmetallic inner sleeve 22 to be inserted through distal end 18 of catheter 12.

The chemical which has been found to have catheter expanding capabilities and is the chemical employed in the preferred embodiment of the chemical soak method is freon. Freon is naturally a liquid at room temperature, but has a high vapor pressure (affinity to become gas). It is the application of this liquid which thereby causes the walls of catheter 12 to swell as the freon is absorbed.

It is the high vapor pressure of freon that allows catheter 12 to return to its normal size. The freon contained in the walls of catheter 12 evaporates, and the swelling is reduced. The use of freon to alter the size of the diameter of catheter 12 is beneficial because freon does not leave a residue upon evaporation.

Freon is not the only chemical which has catheter expanding capabilities, and the present invention is not intended to comprise only freon. It has been noted that cyclohexanone, methyl ethyl ketone, and isopropyl alcohol may also be employed in the present invention. The foregoing are provided as examples, and are not meant to limit the scope of the chemicals having catheter expanding capabilities that may be employed in the present invention.

As illustrated in Figure 2, freon 48 is placed in a container 50 and then a portion of catheter 12 is exposed to freon 48. In one embodiment of the present invention, catheter 12 is exposed to freon 48 for a range of about one to about ten minutes. In the preferred embodiment of the present invention, catheter 12 is exposed to freon 48 for at least about five minutes.

Figure 3 is meant to illustrate the effect the chemical treatment has upon catheter 12 after exposure to freon 48. Expanded portions 52 of catheter 12 exposed to the chemical treatment are enlarged in relation to the portions of catheter 12 not exposed to the chemical treatment. The chemical is absorbed in the walls of catheter 12, and results in swelling. Because the body of catheter 12 is expanded in diameter, lumen 14 of catheter 12 is likewise increased in diameter to a diameter greater than nonmetallic inner sleeve 22.

Now that lumen 14 of expanded portion 52 of catheter 12 immersed in the chemical treatment (preferably freon) is capable of accepting nonmetallic inner sleeve 22, another step is performed. In one preferred method, nonmetallic inner sleeve 22 is advanced through lumen 14 of expanded portion 52 of catheter 12 immersed in freon 48 to a site-selective position which will experience compressive forces 32. Nonmetallic inner sleeve 22 having an outer diameter greater than the inner diameter of catheter 12 before catheter 12 is exposed to a chemical compound and nonmetallic inner sleeve 22 is advanced at least a portion of the way through lumen 14 of catheter 12.

In the preferred embodiment, nonmetallic inner sleeve 22 is placed on a rod 54 as shown in Figure 4. Then rod 54, and therefore nonmetallic inner sleeve 22, are advanced within lumen 14 of catheter 12. The direction of the insertion is indicated by arrow B of Figure 4.

Because the expansive nature of catheter 12 allows nonmetallic inner sleeve 22 to be inserted within catheter 12, nonmetallic inner sleeve 22 can be advanced by rod 54 throughout exposed portion 52 of catheter 12 until nonmetallic inner sleeve 22 becomes positioned at the selected site. In Figure 5, nonmetallic inner sleeve 22 is inserted through lumen 14 of catheter 12 to a site-selected position, however, nonmetallic inner sleeve 22 is not able to become disposed in that portion of catheter 12 that has not been immersed in freon 48. That portion of catheter 12 that has not been immersed in freon 48 has an inner diameter that is smaller than the outer diameter of nonmetallic inner sleeve 22.

As also shown in Figure 5, once nonmetallic inner sleeve 22 has been positioned at a desired site, rod 54 may be withdrawn from nonmetallic inner sleeve 22 in the direction shown by arrow C in Figure 5. Nonmetallic inner sleeve 22 is thereafter in the site selected position which is subject to compressive forces 32.

Finally, the chemical soak method comprises the step of drying expanded portions 52 of catheter 12 exposed to the chemical compound to return expanded portions 52 of catheter 12 to their original size before exposure and leaving nonmetallic inner sleeve 22 within lumen 14 of catheter 12. Catheter 12 having nonmetallic inner sleeve 22 within lumen 14 of catheter 12 is thereafter reinforced against compressive forces 32 to prevent possible partial or total separation of catheter 12.

As the chemically exposed areas of catheter 12 interact with the air, the portions of catheter 12 exposed to freon 48 gradually return to their original size. This result occurs because of the high vapor pressure of the freon. Because of the affinity of the freon to become gas, the freon quickly evaporates and travels from the walls of catheter 12 which are thereby reduced in swelling.

Because the original size of the inner diameter of catheter 12 is smaller than the size of the outer diameter of nonmetallic inner sleeve 22, catheter 12 presses against and fixedly secures nonmetallic inner sleeve 22 within lumen 14 of catheter 12. The structure of the now completed reinforced catheter 10 is illustrated in Figures 1 and 6.

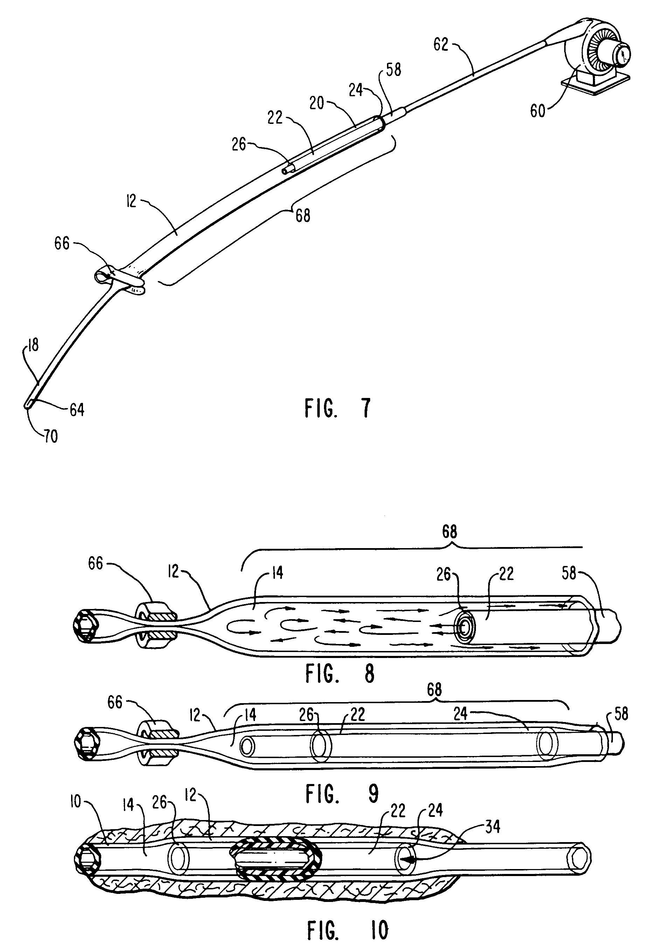

These are the steps which take place in the chemical soak method for preparing reinforced catheter 10. However, the present invention can also be made by a pressurized gas method which can be understood with reference to Figure 7. This method comprises advancing nonmetallic inner sleeve 22, placed on a hollow rod 58 which is connected to a pressurizing gas source 60, through lumen 14 of catheter 12 having distal end 18 capable of being inserted within a patient and a proximal end 20.

As evidenced by Figure 7, the end of hollow rod 58, remote from nonmetallic inner sleeve 22, is connected to low volume, high pressure gas source 60 via an air line 62. In this way, pressurized air can be directed through the air line 62 out the end of hollow rod 58. It should be noted, however, that air is not the only gas which may be directed under pressure through hollow rod 58, other gases may be equally employed.

In the preferred embodiment of the present invention, a range from about 5.4 to 6.2 atmospheres (about 80-90 psi) of gas is introduced to expand lumen 14 of catheter 12.

At this point, it should also be mentioned that the present invention comprises the further step of soft-clamping catheter 12 at a position intermediate the desired reinforcement site and the opening 64 at distal end 18 of catheter 12. Because catheter 12 has an opening at distal end 18 of catheter 12, should air be introduced within lumen 14 at proximal end 20 of catheter 12, air would escape through the opening. In this case, pressurized air passing through hollow rod 58 could not inflate and slightly enlarge catheter 12 to allow nonmetallic inner sleeve 22 to be advanced within catheter 12.

Soft-clamp 66 is illustrated in Figure 7. The clamp is referred to as "soft" in light of its structural feature. Whereas most clamps have angled edges which can possibly injure a catheter, soft-clamp 66 has rounded edges which provide a short-term compressed effect on catheter 12 to block lumen 14. Blocked lumen 14 enables hollow rod 58 to inflate and slightly enlarge a portion of catheter 12.

The method also comprises the step of advancing nonmetallic inner sleeve 22 to an area of catheter 12 experiencing compressive forces 32, catheter 12 expanding in response to the introduction of the pressurized air to allow the advancement of nonmetallic inner sleeve 22. The outer diameter of nonmetallic inner sleeve 22 being greater than the inner diameter of catheter 12 before catheter 12 is expanded. Nonmetallic inner sleeve 22 is advanced at least a portion of the way through catheter 12.

As indicated by Figure 8, the end of hollow rod 58 with nonmetallic inner sleeve 22 mounted thereon, is entered into and advanced along lumen 14 of catheter 12 from open proximal end 20 thereof. The pressurized air passing through hollow rod 58 inflates and slightly enlarges a portion 64 of catheter 12. Enlarged portion 68 of catheter 12 creates an enlarged lumen through which nonmetallic inner sleeve 22 may be passed.

The areas through which pressurized air passes can be shown by arrows in Figure 8. The pressurized air passing through hollow rod 58 inflates and slightly enlarges catheter 12 and escapes therefrom between advancing nonmetallic inner sleeve 22 and the inner wall of catheter 12. As a result, nonmetallic inner sleeve 22 and hollow rod 58 are easily advanced into position as indicated by Figure 9.

According to the pressurized gas method, air at a high pressure and controlled volume is utilized to partially inflate catheter 12 while at the same time providing a nontoxic, low-friction interface layer between nonmetallic inner sleeve 22 and the inner surface of lumen 14 of catheter 12.

In one embodiment of the present invention, a catheter having a Groshong® valve 70 may be employed. Because this type of valve is responsive to pressure differentials, there is a possibility that the Groshong valve could be damaged by the pressurized gas required by the present invention. Therefore, as previously discussed, it is preferred that the present invention comprise the further step of soft-clamping catheter 12 at a position intermediate the desired reinforcement site and the openings at distal end 18 of catheter 12.

Once catheter 12 has been soft-clamped, hollow rod 58 used to advance nonmetallic inner sleeve 22 to a site-selective position in catheter 12 is withdrawn from nonmetallic inner sleeve 22 and catheter 12. Nonmetallic inner sleeve 22 is left within catheter 12 which returns to a normal size after hollow rod 58 has been removed. Catheter 12 having nonmetallic inner sleeve 22 within lumen 14 of catheter 12 is reinforced against compressive forces 32 to prevent partial separation to allow for the extravasation or total separation leading to embolization.

Either the rod 54 (see Figures 4 to 6) or the hollow rod 58 (see Figures 8 to 10) may be equipped with a sliding ring or cylinder which may be employed to aid withdrawal of the rod 54 or 58 from engagement with the sleeve 22.

In one embodiment of the present invention, nonmetallic inner sleeve 22 is advanced within lumen 14 of catheter 12 in the range from about 25 mm to about 610 mm (from about one inch to about two feet) from distal end 18 of catheter 12. In the preferred embodiment, however, nonmetallic inner sleeve 22 is advanced within lumen 14 of catheter 12 about 25 mm to about 38 mm (about one and one-half inches) from distal end 18 of catheter 12.

Implantation of reinforced catheter 10 within the body of a patient can be accomplished in the same way other catheter known to the art are implanted. Most often, implantation will involve the use of some type of induction system employing a needle or trocar and an introducer.

From the foregoing, it will be appreciated that the present invention provides apparatus and methods for reducing the susceptibility of implanted catheters via the percutaneous subclavian route to damaging stresses due to body movement by reinforcing site-selectively the walls of the catheter.

Additionally, it will be appreciated that the present invention provides apparatus and methods for reinforcing a catheter disposed in an area subject to damaging stresses due to body movement which can prevent embolization of the implanted catheter by maintaining a connection between stress-induced severed catheter portions should separation or fractionation occur. Frictional engagement between each of the severed catheter portions surrounding the reinforcing inner sleeve acts to maintain the connection between the severed catheter portions.

Also, it will be appreciated that the present invention provides apparatus and methods for reinforcing a catheter disposed in an area subject to damaging stresses due to body movement which will have the capacity to place and use the catheter without a major deviation from currently existing standard practices.

It will also be appreciated that the present invention provides apparatus and methods for reinforcing a catheter disposed in an area subject to damaging stresses due to body movement which will allow the insertion of a reinforcing inner sleeve which will be soft enough to easily follow the normal path of a catheter as well as preventing partial or total catheter separation due to the pinching action of the clavicle and first rib.

Still yet, it will be appreciated that the present invention provides apparatus and methods f or reinforcing a catheter disposed in an area subject to damaging stresses due to body movement which is easy to handle and manipulate by medical personnel and will not harden or deteriorate over time.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description.

## Claims

1. A catheter to be exposed in a localized region thereof to compressive forces generated by the body of a patient when the catheter is indwelling therein, the catheter having a catheter tube (12) made of a compliant medical grade material, said catheter tube having a distal and a proximal end and a longitudinal lumen (14) extending through a substantial length of the catheter tube;
characterised in that a reinforcing means (22) is positioned within the lumen (14) of the catheter tube (12) for site-selectively reinforcing the localized region.

2. A catheter as recited in Claim 1, characterised in that the reinforcing means comprises a nonmetallic, compliable inner tubular sleeve (22) inserted selectively within at least a portion of the lumen (14) of the said catheter tube and being capable of withstanding compressive forces generated by the body of a patient.

3. A catheter as recited in Claim 2, characterised in that said tubular sleeve (22) is positioned in the lumen (14) of the catheter tube in a range from about 25mm to about 210mm (from about one inch to about two feet) from the distal end (18) of said catheter tube.

4. A catheter as recited in any one of Claims 1-3, characterised in that when the catheter tube (12) is implanted percutaneously in the body of a patient, said catheter tube (12) is so structured as to avoid rupture of sensitive body tissues when being thusly implanted.

5. A catheter as recited in any one of Claims 1-4, characterised in that said catheter is a percutaneously placed indwelling central venous access catheter.

6. A catheter as recited in any one of Claims 1-5, characterised in that said catheter tube is comprised of silicone rubber and has a diameter in a range from about 1.27 mm to about 1.67 mm, wherein the indwelling catheter tube transverses the area between the clavicle and first rib of the patient.

7. An apparatus for site-selectively reinforcing a region of a catheter as recited in Claim 6, characterised in that the localized region of said catheter is subjected to compressive forces generated by the area between the clavicle and first rib of the patient.

8. A catheter as recited in Claims 2-7, characterised in that said tubular sleeve has a diameter in a range from about 1.80 mm to about 1.96 mm (from about 0.071 inches to about 0.077 inches).

9. A catheter, as recited in any one of Claims 2-8, characterised in that said tubular sleeve is made of soft, medical grade silicone rubber and has a length in a range of from about 200 mm to about 216 mm (from about 8 inches to about 8.5 inches).

10. A catheter as recited in any one of Claims 1-9, characterised in that said catheter tube comprises either a single lumen or a plurality of lumens.

11. A catheter as recited in any one of Claims 1-10, characterised in that there is a two-way valve at the distal end (18) of the catheter tube.

12. A catheter as recited in any one of Claims 1-11, characterised in that said catheter tube (12) has a lumen (14) having a valve capable of selectorally affording the ingress or the egress of fluids from the lumen of the catheter tube.

13. A catheter as recited in any one of Claims 1-12, wherein said catheter is constructed so as to be capable of percutaneous implantation within the body of a patient for a time period in a range of from about 10 minutes to about 2 years.

14. A catheter as recited in any one of Claims 1-13, wherein said catheter tube is so constructed that it will not embolize due to fractures in said catheter tube at the localized region of the catheter.

15. A method for site-selectively reinforcing a localized portion of a catheter to be exposed at the localized portion to compressive forces generated by the body of a patient when the catheter is indwelling therein, the catheter having first and second ends and a lumen extending from a first portion of the length of the catheter from the first end thereof to at least the localized portion, the method being characterized by the steps of:
(a) causing the inner diameter of the lumen (14) in the first portion of the catheter to increase from the original inner diameter thereof to an enlarged inner diameter;
(b) advancing a reinforcing sleeve (22) through the lumen in said first portion of said catheter to the localized region thereof, said reinforcing sleeve having an outer diameter greater than the original inner diameter of the lumen in the first portion of said catheter and less than said enlarged inner diameter of said lumen;
(c) leaving said reinforcing sleeve (22) in said lumen at the localized region; and
(d) allowing the inner diameter of said lumen in said first portion of said catheter to return from enlarged inner diameter thereof to the original inner diameter thereof, thereby to secure said reinforcing sleeve (22) within the lumen (14) of said catheter at the localized region to reinforce said catheter against the compressive forces applied at the localized region.

16. A method as recited in Claim 15, characterised in that said step of causing the inner diameter of the lumen (14) in the first portion of the catheter to increase comprises the step of exposing said first portion of the catheter to a chemical compound which causes said first portion of said catheter tube to swell.

17. A method as recited in any one of Claims 15 or 16, characterised in that said first portion of said catheter is immersed in a chemical compound comprising freon.

18. A method as recited in any one of Claims 15-17, characterised in that said reinforcing sleeve (22) is advanced through the lumen in said first portion of said catheter using an advancement rod (54).

19. A method as recited in any one of Claims 15-17, characterised in that the method further comprises the step of soft clamping said catheter tube on the opposite side of the localized region from said first end thereof prior to the step of advancing said reinforcing sleeve.

20. A method as recited in claim 15, characterised in that said step of causing the inner diameter of the lumen (14) in the first portion of the catheter to increase comprises the steps of:
(a) soft clamping the catheter on the opposite side of the localized region from said first end thereof;
(b) securing a hollow reinforcing sleeve (22) to one end of a hollow advancement rod (58);
(c) connecting the other end of said hollow advancement rod to a low volume, high pressure gas source; and
(d) inserting said reinforcing sleeve (22) on said advancement rod (58) into the lumen of said catheter from said first end thereof, the inner diameter of the lumen of the catheter expanding due to the introduction thereto of pressurized gas through said hollow rod and said reinforcing sleeve.

21. A method as recited in Claim 20, characterised in that gas at a pressure in a range from about 5.4 to about 6.2 atmospheres (from about 80 psi to about 90 psi) is introduced through the hollow advancement rod and the reinforcing sleeve to expand the lumen of the catheter.

22. A method as recited in any one of Claims 18 to 21, characterised in that a ring or cylinder is attached to said advancement rod to assist in the placement of the reinforcing sleeve at the localized portion of the catheter or to assist in withdrawing the rod (54, 58) from engagement with the sleeve 22.

23. A method as recited in any one of Claims 15 to 22, characterised in that said reinforcing sleeve has an outer diameter in a range of from about 1.8 mm to about 1.96 mm (from about 0.071 inches to about 0.077 inches) and the original inner diameter of the lumen of the catheter tube is in a range of from about 1.4 mm to about 1.7 mm (from about 0.055 inches to about 0.066 inches).

24. A method as recited in any one of Claims 16-24, characterised in that the localized region to which the reinforcing sleeve is advanced corresponds to the area between the clavicle and first rib of a patient when the catheter is indwelling therein.

## Patentansprüche

1. Katheter, der in einem begrenzten Bereich desrselben Druckkräften ausgesetzt werden soll, die durch den Körper eines Patienten erzeugt werden, wenn der Katheter darin verweilt, wobei der Katheter einen Katheterschlauch (12) aufweist, der aus einem nachgiebigen, für medizinische Zwecke geeignetem Material hergestellt ist, wobei der Katheterschlauch ein distales und ein proximales Ende und einen länglichen Hohlraum (14) aufweist, der sich über eine beträchtliche Länge des Katheterschlauchs erstreckt;
dadurch **gekennzeichnet,** daß eine Verstärkungseinrichtung (22) in dem Hohlraum (14) des Katheterschlauchs (12) angeordnet ist, um den begrenzten Bereich an einer wählbaren Stelle zu verstärken.

2. Katheter nach Anspruch 1, dadurch gekennzeichnet, daß die Verstärkungseinrichtung eine nichtmetallische, nachgiebige, innere röhrenförmige Hülse (22) aufweist, die ausgewählt innerhalb wenigstens eines Abschnitts des Hohlraums (14) des Katheterschlauchs eingeführt ist, und die Druckkräften widerstehen kann, die durch den Körper eines Patienten erzeugt werden.

3. Katheter nach Anspruch 2, dadurch gekennzeichnet, daß die röhrenförmige Hülse (22) in dem Hohlraum (14) des Katheterschlauchs in einem Bereich von etwa 25 mm bis etwa 210 mm (von etwa 1 Inch bis etwa 2 Fuß) von dem distalen Ende (18) des Katheterschlauchs angeordnet ist.

4. Katheter nach einem der Ansprüche 1 bis 3, dadurch gekennzeichnet, daß, wenn der Katheterschlauch (12) durch die Haut hindurch in den Körper eines Patienten implantiert wird, der Katheterschlauch (12) derart aufgebaut ist, daß ein Reißen von empfindlichen Körpergeweben vermieden wird, wenn er derart implantiert wird.

5. Katheter nach einem der Ansprüche 1 bis 4, dadurch gekennzeichnet, daß der Katheter ein durch die Haut hindurch eingesetzter, verweilender zentraler Venenkatheter ist.

6. Katheter nach einem der Ansprüche 1 bis 5, dadurch gekennzeichnet, daß der Katheterschlauch aus Silikongummi besteht und einen Durchmesser in einem Bereich von etwa 1,27 mm bis etwa 1,67 mm aufweist, wobei der verweilende Katheterschlauch den Bereich zwischen dem Schlüsselbein und der ersten Rippe des Patienten durchquert.

7. Vorrichtung zum Verstärken eines Bereichs eines Katheters an einer wählbaren Stelle nach Anspruch 6, dadurch gekennzeichnet, daß der begrenzte Bereich des Katheters Druckkräften unterliegt, die durch den Bereich zwischen dem Schlüsselbein und der ersten Rippe des Patienten erzeugt werden.

8. Katheter nach den Ansprüchen 2 bis 7, dadurch gekennzeichnet, daß die röhrenförmige Hülse einen Durchmesser in einem Bereich von etwa 1,80 mm bis etwa 1,96 mm (von etwa 0,071 Inch bis etwa 0,077 Inch) aufweist.

9. Katheter nach einem der Ansprüche 2 bis 8, dadurch gekennzeichnet, daß die röhrenförmige Hülse aus weichem, für medizinische Zwecke geeignetem Silikongummi hergestellt ist und eine Länge in einem Bereich von etwa 200 mm bis etwa 216 mm (von etwa 8 Inch bis etwa 8,5 Inch) aufweist.

10. Katheter nach einem der Ansprüche 1 bis 9, dadurch gekennzeichnet, daß der Katheterschlauch entweder einen einzigen Hohlraum oder mehrere Hohlräume aufweist.

11. Katheter nach einem der Ansprüche 1 bis 10, dadurch gekennzeichnet, daß sich an dem distalen Ende (18) des Katheterschlauchs ein Zweiwegeventil befindet.

12. Katheter nach einem der Ansprüche 1 bis 11, dadurch gekennzeichnet, daß der Katheterschlauch (12) einen Hohlraum (14) mit einem Ventil aufweist, das wahlweise den Eintritt oder den Austritt von Flüssigkeiten in den oder aus dem Hohlraum des Katheterschlauchs gewähren kann.

13. Katheter nach einem der Ansprüche 1 bis 12, wobei der Katheter derart aufgebaut ist, daß er durch die Haut hindurch in den Körper eines Patienten für eine Zeitdauer in einem Bereich von etwa 10 Minuten bis etwa 2 Jahren implantiert werden kann.

14. Katheter nach einem der Ansprüche 1 bis 13, wobei der Katheterschlauch derart aufgebaut ist, daß er aufgrund von Brüchen in dem Katheterschlauch in dem begrenzten Bereich des Katheters keine Embolie auslöst.

15. Verfahren zum Verstärken eines begrenzten Abschnitts eines Katheters an einer wählbaren Stelle, der in dem begrenzten Abschnitt Druckkräften ausgesetzt werden soll, die durch den Körper eines Patienten erzeugt werden, wenn der Katheter darin verweilt, wobei der Katheter ein erstes und ein zweites Ende und einen Hohlraum aufweist, der sich von einem ersten Abschnitt der Länge des Katheters von dessen erstem Ende wenigstens bis zu dem begrenzten Abschnitt erstreckt, wobei das Verfahren gekennzeichnet ist durch die Schritte:
(a) Veranlassen einer Vergrößerung des inneren Durchmessers des Hohlraums (14) in dem ersten Abschnitt des Katheters von seinem ursprünglichen inneren Durchmesser auf einen vergrößerten inneren Durchmesser;
(b) Vorschieben einer verstärkenden Hülse (22) durch den Hohlraum in dem ersten Abschnitt des Katheters zu dem begrenzten Bereich, wobei die verstärkende Hülse einen äußeren Durchmesser aufweist, der größer ist als der ursprüngliche innere Durchmesser des Hohlraums in dem ersten Abschnitt des Katheters und geringer ist als der vergrößerte innere Durchmesser des Hohlraums;
(c) Zurücklassen der verstärkenden Hülse (22) in dem Hohlraum in dem begrenzten Bereich; und
(d) Ermöglichen eines Rückkehrens des inneren Durchmessers des Hohlraums in dem ersten Abschnitt des Katheters von dem vergrößerten inneren Durchmesser zu dem ursprünglichen inneren Durchmesser, wodurch die verstärkende Hülse (22) innerhalb des Hohlraums (14) des Katheters in dem begrenzten Bereich derart gesichert wird, daß sie den Katheter gegen die Druckkräfte verstärkt, die in dem begrenzten Bereich aufgebracht werden.

16. Verfahren nach Anspruch 15, dadurch gekennzeichnet, daß das Veranlassen einer Vergrößerung des inneren Durchmessers des Hohlraums (14) in dem ersten Abschnitt des Katheters den Schritt aufweist: der erste Abschnitt des Katheters wird einer chemischen Verbindung ausgesetzt, die den ersten Abschnitt des Katheterschlauchs anschwellen läßt.

17. Verfahren nach einem der Ansprüche 15 oder 16, dadurch gekennzeichnet, daß der erste Abschnitt des Katheters in eine chemische Verbindung, die Freon aufweist, eingetaucht wird.

18. Verfahren nach einem der Ansprüche 15 bis 17, dadurch gekennzeichnet, daß die verstärkende Hülse (22) durch den Hohlraum in dem ersten Abschnitt des Katheters unter Verwendung einer Vorschubstange (54) vorgeschoben wird.

19. Verfahren nach einem der Ansprüche 15 bis 17, dadurch gekennzeichnet, daß das Verfahren ferner den Schritt aufweist: vor dem Schritt des Vorschiebens der verstärkenden Hülse wird der Katheterschlauch an der Seite des begrenzten Bereichs, die dem ersten Ende gegenüberliegt, weich geklammert.

20. Verfahren nach Anspruch 15, dadurch gekennzeichnet, daß das Veranlassen einer Vergrößerung des inneren Durchmessers des Hohlraums (14) in dem ersten Abschnitt des Katheters folgende Schritte aufweist:
(a) Weiches Klammern des Katheters an der Seite des begrenzten Bereichs, die dem ersten Ende gegenüberliegt;
(b) Befestigen einer hohlen verstärkenden Hülse (22) an einem Ende einer hohlen Vorschubstange (58);
(c) Verbinden des anderen Endes der hohlen Vorschubstange mit einer Gasquelle mit niedrigem Volumen und hohem Druck; und
(d) Einführen der verstärkenden Hülse (22) auf der Vorschubstange (58) in den Hohlraum des Katheters von dessen erstem Ende aus, wobei sich der innere Durchmesser des Hohlraums des Katheters wegen der Einführung von Druckgas durch die hohle Stange und die verstärkende Hülse ausdehnt.

21. Verfahren nach Anspruch 20, dadurch gekennzeichnet, daß Gas bei einem Druck in einem Bereich von etwa 5,4 bis etwa 6,2 Atmosphären (von etwa 80 psi bis etwa 90 psi) durch die hohle Vorschubstange und die verstärkende Hülse eingeführt wird, um den Hohlraum des Katheters auszudehnen.

22. Verfahren nach einem der Ansprüche 18 bis 21, dadurch gekennzeichnet, daß ein Ring oder ein Zylinder an der Vorschubstange derart angebracht ist, daß er die Anordnung der verstärkenden Hülse in dem begrenzten Bereich des Katheters unterstützt, oder das Zurückziehen der Stange (54, 58) von dem Eingriff mit der Hülse (22) unterstützt.

23. Verfahren nach einem der Ansprüche 15 bis 22, dadurch gekennzeichnet, daß die verstärkende Hülse einen äußeren Durchmesser in einem Bereich von etwa 1,8 mm bis etwa 1,96 mm (von etwa 0,071 Inch bis etwa 0,077 Inch) aufweist, und der ursprüngliche innere Durchmesser des Hohlraums des Katheterschlauchs in einem Bereich von etwa 1,4 mm bis etwa 1,7 mm (von etwa 0,055 Inch bis etwa 0,066 Inch) liegt.

24. Verfahren nach einem der Ansprüche 16 bis 23, dadurch gekennzeichnet, daß der begrenzte Bereich, zu dem die verstärkende Hülse vorgeschoben wird, dem Bereich zwischen dem Schlüsselbein und der ersten Rippe eines Patienten entspricht, wenn der Katheter darin verweilt.

## Revendications

1. Cathéter devant être exposé dans une zone localisée de celui-ci à des forces de compression produites par le corps d'un malade lorsque le cathéter est placé intérieurement, le cathéter ayant un tube de cathéter (12) constitué d'un matériau élastique de la qualité médicale, ledit tube du cathéter ayant une extrémité distale et une extrémité proximale et un passage longitudinal (14) s'étendant à travers un tronçon important du tube de cathéter;
caractérisé en ce qu un moyen de renforcemnt (22) est placé à l'intérieur du passage (14) du tube de cathéter (12) pour renforcer d'une manière sélective en matière d'endroit la zone localisée.

2. Cathéter selon la revendication 1, caractérisé en ce que le moyen de renforcement comprend un manchon tubulaire intérieur non métallique, élastique (22) inséré sélectivement dans au moins une portion du passage (14) dudit tube de cathéter et capable de supporter les forces de compression produites par le corps d'un malade.

3. Cathéter selon la revendication 2, caractérisé en ce que ledit manchon tubulaire (22) est placé dans le passage (14) du tube de cathéter dans une plage d'environ 25 mm à environ 210 mm (d'environ 1 pouce à environ 2 pieds) par rapport à l'extrémité distale (18) dudit tube de cathéter.

4. Cathéter selon l'une des revendications 1 à 3, caractérisé en ce que, lorsque le tube de cathéter (12) est implanté de façon percutanée dans le corps d'un malade, ledit tube de cathéter (12) est structuré de façon à éviter une rupture des tissus sensibles du corps lorsqu'il est ainsi implanté.

5. Cathéter selon l'une quelconque des revendications 1 à 4, caractérisé en ce que ledit cathéter est un cathéter à accès veineux central interne placé de manière percutanée.

6. Cathéter selon l'une quelconque des revendications 1 à 5, caractérisé en ce que ledit tube de cathéter est constitué d'un caoutchouc de silicone et a un diamètre dans la gamme comprise d'environ 1,27 mm à environ 1,67 mm, où le tube de cathéter interne traverse la zone comprise entre la clavicule et la première côte du malade.

7. Appareil pour le renforcement, sélectif en matière d'endroit, d'une zone d'un cathéter selon la revendication 6, caractérisé en ce que la zone localisée dudit cathéter est soumise à des forces de compression produites par la zone comprise entre la clavicule et la première côte du malade.

8. Cathéter selon les revendications 2 à 7, caractérisé en ce que ledit manchon tubulaire a un diamètre dans la gamme comprise d'environ 1,80 mm à environ 1,96 mm (d'environ 0,071 pouce à environ 0,077 pouce).

9. Cathéter selon l'une quelconque des revendications 2-8, caractérisé en ce que ledit manchon tubulaire est constitué d'un caoutchouc de silicone tendre, de la qualité médicale, et a une longueur dans la gamme comprise d'environ 200 mm à environ 216 mm (d'environ 8 pouces à environ 8,5 pouces).

10. Cathéter selon l'une quelconque des revendications 1-9, caractérisé en ce que ledit tube de cathéter comprend soit un seul passage soit une multitude de passages.

11. Cathéter selon l'une quelconque des revendications 1-10, caractérisé en ce qu'il y a une soupape bidirectionnelle à l'extrémité distale (18) du tube de cathéter.

12. Cathéter selon l'une quelconque des revendications 1-11, caractérisé en ce que ledit tube de cathéter (12) comporte un passage (14) ayant une soupape capable de permettre de façon sélective l'entrée ou la sortie par secteur de fluides à partir du passage du tube de cathéter.

13. Cathéter selon l'une quelconque des revendications 1-12, dans lequel ledit cathéter est construit de manière à pouvoir être implanté de façon percutanée à l'intérieur du corps d'un patient pendant une durée comprise d'environ 10 minutes à environ 2 ans.

14. Cathéter selon l'une quelconque des revendications 1-13, dans lequel le tube de cathéter est construit de façon à ne pas provoquer une embolisation à cause de fractures dans le tube de cathéter à la zone localisée du cathéter.

15. Procédé pour renforcer d'une façon sélective en matière d'endroit une portion localisée d'un cathéter destiné à être exposé au droit de la portion localisée à des forces de compression produites par le corps d'un malade lorsque le cathéter y est placé, le cathéter ayant des première et seconde extrémités et un passage s'étendant entre une première portion du tronçon du cathéter à partir de sa première extrémité et au moins la portion localisée, le procédé étant caractérisé par les étapes consistant à :
(a) faire en sorte que le diamètre intérieur du passage (14) dans la première portion du cathéter augmente à partir de son diamètre intérieur d'origine jusqu'à un diamètre intérieur agrandi;
(b) faire avancer un manchon de renforcement (22) à travers le passage dans ladite première portion dudit cathéter jusqu'à sa zone localisée, ledit manchon de renforcement ayant un diamètre extérieur supérieur au diamètre intérieur d'origine du passage dans la première portion dudit cathéter et inférieur audit diamètre intérieur agrandi dudit passage;
(c) laisser ledit manchon de renforcement (22) dans ledit passage à la zone localisée; et
(d) permettre au diamètre intérieur dudit passage dans ladite première portion dudit cathéter de revenir de son diamètre intérieur agrandi à son diamètre intérieur d'origine, d'où il résulte la fixation dudit manchon de renforcement (22) à l'intérieur du passage (14) dudit cathéter à la zone localisée pour renforcer ledit cathéter contre les forces de compression appliquées à la zone localisée.

16. Procédé selon la revendication 15, caractérisé en ce que ladite étape consistant à faire en sorte que le diamètre intérieur du passage (14) dans la première portion du cathéter augmente comprend l'étape consistant à exposer ladite première portion du cathéter à un composé chimique qui provoque le gonflement de ladite première portion dudit tube de cathéter.

17. Procédé selon l'une quelconque des revendications 15 ou 16, caractérisé en ce que ladite première portion dudit cathéter est immergée dans un composé chimique comprenant du fréon.

18. Procédé selon l'une quelconque des revendications 15-17, caractérisé en ce que ledit manchon de renforcement (22) est avancé dans le passage dans ladite première portion dudit cathéter en utilisant une tige d'avancement (54).

19. Procédé selon l'une quelconque des revendications 15-17, caractérisé en ce que le procédé comprend en outre l'étape consistant à fixer doucement ledit tube de cathéter sur le côté opposé de la zone localisée par rapport à sadite première extrémité avant l'étape consistant à faire avancer ledit manchon de renforcement.

20. Procédé selon la revendication 15, caractérisé en ce que ladite étape consistant à faire en sorte que le diamètre intérieur du passage (14) dans la première portion du cathéter augmente comprend les étapes consistant à :
(a) fixer doucement le cathéter sur le côté opposé de la zone localisée par rapport à sadite première extrémité;
(b) fixer un manchon de renforcement creux (22) à une extrémité d'une tige creuse d'avancement (58);
(c) relier l'autre extrémité de ladite tige creuse d'avancement à une source de gaz à haute pression, faible volume; et
(d) insérer ledit manchon de renforcement (22) sur ladite tige d'avancement (58) dans le passage dudit cathéter à partir de sadite première extrémité, le diamètre intérieur du passage du cathéter s'étendant sous l'effet de l'introduction dans celui-ci d'un gaz sous pression par l'intermédiaire de ladite tige creuse et dudit manchon de renforcement.

21. Procédé selon la revendication 20, caractérisé en ce que le gaz à une pression comprise d'environ 5,4 à environ 6,2 atmosphères (d'environ 80 psi à environ 90 psi) est introduit par l'intermédiaire de la tige creuse d'avancement et du manchon de renforcement pour dilater le passage du cathéter.

22. Procédé selon l'une quelconque des revendications 18 à 21, caractérisé en ce qu'une bague ou un cylindre est fixé à ladite tige d'avancement pour aider au placement du manchon de renforcement à la portion localisée du cathéter ou pour aider à l'extraction de la tige (54, 58) pour l'enlever du manchon (22).

23. Procédé selon l'une quelconque des revendications 15 à 22, caractérisé en ce que ledit manchon de renforcement a un diamètre extérieur dans la gamme d'environ 1,8 mm à environ 1,96 mm (d'environ 0,071 pouce à environ 0,077 pouce) et le diamètre intérieur d'origine du passage du tube de cathéter se trouve dans la gamme d'environ 1,4 mm à environ 1,7 mm (d'environ 0,055 pouce à environ 0,066 pouce).

24. Procédé selon l'une quelconque des revendications 16-23, caractérisé en ce que la zone localisée dans laquelle le manchon de renforcement est avancé correspond à la zone séparant la clavicule et la première côte d'un malade lorsque le cathéter se trouve à l'intérieur de celle-ci.
